# EUROPÄISCHE PATENTSCHRIFT

(11) **EP 3 056 172 B1**
(45) Veröffentlichungstag und Bekanntmachung des Hinweises auf die Patenterteilung: **04.09.2019**
(21) Anmeldenummer: 15153126.6
(22) Anmeldetag: 29.01.2015
(51) Int. Cl.: A61F 2/46, A61B 90/00

(54) **Instrument zur Behandlung der Wirbelsäule**
Instrument for treatment of the spinal column
Instrument de traitement de colonne vertébrale

(43) Veröffentlichungstag der Anmeldung: 17.08.2016
(73) Patentinhaber: Silony Medical International AG, 8500 Frauenfeld (CH)
(72) Erfinder: Heuer, Frank, 70794 Filderstadt (DE); Fromm, Michael, 72537 Mehrstetten (DE); Carboniero, Emanuele, 73732 Esslingen (DE)
(74) Vertreter: Stolmár & Partner Patentanwälte PartG mbB

(56) Entgegenhaltungen:
- DE-U1-202005 018 655
- DE-U1-202010 011 948
- US-A1- 2006 084 986
- US-A1- 2008 243 131
- US-A1- 2010 280 620

## Beschreibung

### Gebiet der Erfindung

Die Erfindung betrifft ein Instrument zum Implantieren eines Wirbelsäulenstützelements, insbesondere ein Cage oder ein Wirbelkörperersatzimplantat.

### Stand der Technik

Wirbelsäulenstützelemente werden zur Unterstützung der Wirbelsäule beispielsweise bei einem Bandscheibenvorfall verwendet. Dazu wird beispielsweise ein Cage an die dafür vorgesehene Stelle zwischen zwei Wirbelkörpern eingesetzt, um einen definierten Abstand zwischen den Wirbelkörpern einzustellen und eine knöcherne Fusion mechanisch zu begünstigen. Eine andere Anwendung solcher Wirbelsäulenstützelemente kann auch der Wirbelkörperersatz sein. Dabei kann ein kompletter Wirbel durch ein Wirbelsäulenstützelement ersetzt werden.

Um eine genaue Positionierung zu ermöglichen und vor allem zu verhindern, dass der Cage oder das Wirbelkörperersatzimplantat aus Versehen zu weit in die Wirbelsäule hineingeschoben wird und dabei Verletzungen entstehen, ist an einigen Instrumenten ein Anschlag vorgesehen, der mit einem der benachbarten Wirbelkörper in Kontakt gerät und so dem Operateur eine Rückmeldung gibt, dass der Cage in der richtigen Position ist. So wird verhindert, dass der Cage auch weiter in die Wirbelsäule hineingeschoben werden kann.

Ein Problem ist nun, dass der Körperbau der Patienten unterschiedlich ist, weswegen Cages von verschiedener Größe verwendet werden. Da der Anschlag an einem Instrument jedoch nicht für verschieden große Cages optimal ausgelegt ist, kann dies unter Umständen zu Problemen führen, beispielsweise weil der Anschlag kein weiteres Einschieben des Cages erlaubt, obwohl dieser noch nicht an der richtigen Position sitzt, oder dass der Anschlag erst mit der Wirbelsäule in Kontakt gerät, wenn der Cage zu weit in die Wirbelsäule geschoben wurde. Aus diesem Grund werden für jeden Cage eigene Instrumente angeboten, die für die spezielle Größe optimiert wurden.

Die US 2010/280620 offenbart eine Probeimplantatanordnung. Sie umfasst ein Implantat und ein Instrument zum Einführen des Implantats. Das Instrument umfasst einen Gewindeschaft mit einem zu einem distalen Ende benachbarten Außengewinde. Das Implantat ist mit einem Innengewinde versehen und an dem distalen Ende des Schafts befestigbar. Der Gewindeschaft weist ein erstes Befestigungsmittel auf. Das Instrument umfasst weiterhin einen Abstandhalter mit einem Anschlag und einem zum ersten Befestigungsmittel komplementären zweiten Befestigungsmittel. Die Befestigungsmittel sind voneinander lösbar und legen den Abstandhalter in proximaler Axialrichtung fest.

Die DE 20 2005 018 655 U1 offenbart ein Chirurgisches Führungsinstrument für ein zum Bearbeiten von Wirbelkörpern ausgebildetes und ein distales Werkzeugende aufweisendes Bearbeitungswerkzeug, wobei das Führungsinstrument einen im Wesentlichen langgestreckten, ein proximales und ein distales Ende aufweisenden und eine Längsachse definierenden Instrumentenkörper umfasst, der eine Führungsvorrichtung für das Bearbeitungswerkzeug trägt, dadurch gekennzeichnet, dass die Führungsvorrichtung derart angeordnet und ausgebildet ist, dass mit ihr das Bearbeitungswerkzeug entlang einer von der Führungsvorrichtung definierten Bewegungsbahn zwangsführbar ist und dass die Bewegungsbahn einer überlagerten Translations-Schwenk-Bewegung entspricht. Das Führungsinstrument weist ein Implantat auf.

Die US 2008/243131 offenbart ein Wirbelsäulenimplantat-Einsatzwerkzeug mit einem Schaft, der ein proximales Ende mit einem Griff und ein distales Ende aufweist, das zum Halten eines Implantats geeignet ist, mit einem verstellbaren Anschlag, der gleitend auf dem Schaft des Werkzeuges sitzt, wobei der einstellbare Anschlag ein gegabeltes distales Ende aufweist.

### Darstellung der Erfindung

Aufgabe der Erfindung ist es nun, ein Instrument zum Implantieren eines Wirbelsäulenstützelements bereitzustellen, das für unterschiedlich große Wirbelsäulenstützelemente geeignet ist.

Ein solches Instrument ist in Anspruch 1 und in Anspruch 14 beschrieben. Weitere, die Erfindung ausgestaltende Merkmale sind in den Unteransprüchen enthalten.

Ein Instrument zum Implantieren eines Wirbelsäulenstützelements (z.B. ein Cage oder ein Wirbelkörperersatzimplantat) umfasst einen Hauptkörper mit einem Griffabschnitt zum Halten und Führen des Instruments und mit einer Lagereinrichtung am distalen Ende des Hauptkörpers zum Lagern des Wirbelsäulenstützelements, wobei der Hauptkörper ein erstes Befestigungsmittel aufweist. Ferner umfasst das Instrument einen Abstandhalter mit zumindest einem Anschlag und einem zum ersten Befestigungsmittel komplementären zweiten Befestigungsmittel, wobei das oder die Befestigungsmittel lösbar sind und den Anschlag zumindest in proximaler Axialrichtung festlegen und die Lagereinrichtung sich durch den gesamten Hauptkörper erstreckt und die Lagereinrichtung als Welle ausgebildet ist, die sich schrittweise oder kontinuierlich vom proximalen Ende bis zum distalen Ende verjüngt. In einer anderen Ausgestaltung weist das Instrument zum Implantieren eines Wirbelsäulenstützelements einen Hauptkörper mit einem Griffabschnitt zum Halten und Führen des Instruments und mit einer Lagereinrichtung am distalen Ende des Hauptkörpers zum Lagern des Wirbelsäulenstützelements und einen Abstandhalter mit zumindest einem Anschlag auf, wobei der Abstandhalter in Axialrichtung verstellbar am Hauptkörper befestigt ist und die Lagereinrichtung sich durch den gesamten Hauptkörper erstreckt und die Lagereinrichtung als Welle ausgebildet ist, die sich schrittweise oder kontinuierlich vom proximalen Ende bis zum distalen Ende verjüngt. Durch einen verstellbaren Anschlag oder durch einen lösbaren Anschlag wird ermöglicht, dass verschiedene Wirbelsäulenstützelemente auf dem Instrument implantiert werden können und der Anschlag die richtige Position bestimmen kann, beispielsweise indem er für das jeweilige Wirbelsäulenstützelement eingestellt wird oder indem der Abstandhalter ausgetauscht wird. Insbesondere sind dafür verschiedene Abstandhalter mit verschiedenen Größen zum Austausch vorgesehen. Der Abstandhalter ist vorzugsweise seitlich am, auf oder unter dem Instrument angebracht. "Seitlich", "auf" oder "unter" betrifft dabei die Position des Abstandshalters während der Anwendung des Instruments. Insbesondere kann der Anschlag auch flexibel an den verschiedenen Positionen angebracht werden, d.h. je nach Anwendungserfordernis kann der Anschlag oben, unten oder seitlich an den entsprechenden Befestigungselementen vorgesehen sein. Dadurch hat der Anwender immer freie Sicht auf die Operationsstelle.

Die Befestigungsmittel können als Steckmittel ausgebildet sein. Solche Steckmittel sind sehr einfach voneinander zu lösen und stellen gleichzeitig eine sichere Befestigung des Abstandhalters am Instrument dar.

Der Abstandhalter erstreckt sich insbesondere entlang der Hauptachse des Hauptkörpers und kann weiter vorzugsweise gabelförmig mit zumindest zwei Gabelästen ausgebildet sein, die insbesondere im Wesentlichen parallel verlaufen. Die Stabilität des Abstandhalters insbesondere in Bezug auf den Hauptkörper wird dadurch verbessert, indem das Anstoßen des Anschlags kein oder ein möglichst geringes Moment um das Wirbelsäulenstützelement. erzeugt. Insbesondere können dafür die Gabeläste eines gabelförmig ausgebildeten Abstandhalters symmetrisch zur Mittelachse des Hauptkörpers ausgebildet sein, wenn aus einer Draufsicht auf den montierten Abstandhalter betrachtet.

Die Befestigungsmittel sind insbesondere als Federklemme und Eingriffsabschnitt ausgebildet, wobei die Federklemme und der Eingriffsabschnitt sowohl am Hauptkörper als auch am Abstandhalter ausgebildet sein können. Ein Federkörper und ein entsprechender Eingriffsabschnitt sind einfach herzustellen und gleichzeitig ein zuverlässiges Befestigungsmittel, das den Abstandhalter am Hauptkörper in Längsrichtung festlegen und gleichzeitig flexibel in Umfangsrichtung bleiben kann. Dies kann beispielsweise dann vorteilhaft sein, wenn das Wirbelsäulenstützelement in Umfangsrichtung nicht festgesetzt werden kann und so die Ausrichtung in Umfangsrichtung flexibel ist. Dann kann der Abstandhalter entsprechend in Umfangsrichtung eingestellt werden. Die Federklemmen können Enden aufweisen, die voneinander weg zeigen, wodurch das Befestigen des Abstandhalters am Hauptkörper erleichtert wird.

Vorzugsweise sind mehr erste als zweite Befestigungsmittel oder mehr zweite als erste Befestigungsmittel am Hauptkörper oder am Abstandhalter vorgesehen. So wird ermöglicht, dass der Abstandhalter an verschiedenen Positionen am Hauptkörper anbringbar ist.

Der Hauptkörper des Instruments ist vorzugsweise länglich und insbesondere stabförmig und weist insbesondere einen runden Querschnitt auf. Eine solche Form ist einfacher zu handhaben und flexibler bei der Anwendung, da die Position des Wirbelsäulenstützelements in Umfangsrichtung nicht festgelegt werden muss. Insbesondere kann dies zusammen mit der Federklemme vorteilhaft sein. Der Querschnitt des Hauptkörpers ist aber prinzipiell beliebig

Die Lagereinrichtung des Instruments ist vorzugsweise als Stift, insbesondere als Gewindestift am distalen Ende des Hauptkörpers ausgebildet. Auf diesen ist das Wirbelsäulenstützelement dann aufschraubbar.

Die Lagereinrichtung, beispielsweise der Stift, erstreckt sich insbesondere durch den gesamten Hauptkörper und ist weiter vorzugsweise am proximalen Ende des Hauptkörpers betätigbar. Durch eine solche Konstruktion kann der Stift aus dem Wirbelsäulenstützelement herausgedreht werden, ohne dass der Hauptkörper bewegt werden muss, was die Verletzungswahrscheinlichkeit durch den Hauptkörper verringert. Ferner ist der Stift lösbar im Hauptkörper gelagert. Dadurch kann dieser und auch das ganze Instrument einfacher gereinigt werden. Ferner weist eine solche Lagereinrichtung vorzugsweise eine Sicherung gegen das Herausfallen auf, beispielsweise eine Kappe und/oder eine Sicherung umfassend ein aufeinander abgestimmtes Innen- und Außengewinde. Mit Lagereinrichtung ist insbesondere nicht nur das Element gemeint, das tatsächlich das Wirbelsäulenstützelement lagert, sondern auch das Betätigungselement und die Verbindung zwischen diesen. Die Lagereinrichtung kann dabei einstückig ausgebildet sein oder auch mehrere miteinander verbundene Elementen umfassen.

Weiter vorzugsweise weist das Instrument zwischen Hauptkörper und Wirbelsäulenstützelement eine Rotationshemmung auf, die insbesondere als Zapfen und/oder Nut ausgebildet ist. Diese sind am Wirbelsäulenstützelement und am distalen Ende des Hauptkörpers vorgesehen und verhindern, dass sich das Wirbelsäulenstützelement relativ zum Hauptkörper in Umfangsrichtung drehen kann.

Der Abstandhalter kann ferner auch in Bezug auf den Hauptkörper rotationssicher gelagert bzw. befestigt werden, insbesondere durch Zapfen und/oder Nut, die an Abstandhalter und Hauptkörper ausgebildet sind. Vorzugsweise können eine Vielzahl von solchen rotationshemmenden Elementen in Umfangsrichtung vorgesehen sein.

### Kurze Beschreibung der Figuren

Fig. 1 zeigt ein erfindungsgemäßes Instrument, das aus drei Hauptelementen besteht;
Fig. 2 zeigt ein montiertes erfindungsgemäßes Instrument aus Fig. 1 in einem Querschnitt;
Fig. 3 zeigt das montierte Instrument aus Fig. 1 in einer Seitenansicht;
Fig. 4 zeigt das erfindungsgemäße Instrument aus Fig. 1 in einer Draufsicht; und
Fig. 5 zeigt ein erfindungsgemäßes Instrument in einer isometrischen Explosionsansicht.

### Beschreibung der bevorzugten Ausführungsformen

Im Folgenden beschreibt distal ein zum Patienten gerichtetes Ende des Instruments, während proximal das Ende bezeichnet, das zum Anwender hin zeigt. Ferner beschreibt eine Längsrichtung des Instruments eine Richtung entlang der Längsachse, d.h. vom proximalen bis zum distalen Ende (oder entgegengesetzt), radial eine Richtung senkrecht zur Längsrichtung und Umfangsrichtung eine Richtung entlang des Umfangs des Instruments (um die Längsachse herum).

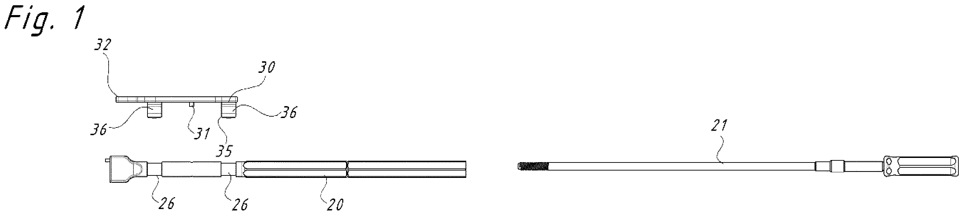

Das Instrument 10 besitzt einen Hauptkörper 20, der sich längs erstreckt. An diesem Hauptkörper 20 ist zumindest ein Griffabschnitt vorgesehen, an dem der Hauptkörper vom Anwender gehalten und geführt werden kann. Am distalen Ende des Instruments ist eine Lagereinrichtung 22 vorgesehen, an der ein Wirbelsäulenstützelement (nicht gezeigt) angebracht werden kann. In Figur 1 ist eine bevorzugte Ausführungsform des Instruments 10 in dessen Einzelteilen dargestellt. Gezeigt sind der Hauptkörper 20, der vorzugsweise als Hohlkörper ausgebildet ist, ein als Welle 21 ausgebildetes Lagerelement 22 und der Abstandhalter 30. Der Hauptkörper ist vorzugsweise im Wesentlichen rund ausgeführt, kann aber auch prinzipiell jede andere Form aufweisen, wie beispielsweise einen polygonalen Querschnitt (drei-, vier-, acht-, zwölfeckig; rechteckförmig) oder einen ovalen Querschnitt. Der Hohlkörper weist am distalen Ende eine in radialer Richtung sich erstreckende Verbreiterung auf, in die beispielsweise der später beschriebene Verdrehsicherungsstift für das Wirbelsäulenstützelement eingesteckt werden kann. Der Griffabschnitt befindet sich insbesondere in der proximalen Hälfte des Instruments, die Befestigungsmittel 26, 36 sind vorzugsweise in der distalen Hälfte angeordnet.

In den Figuren ist diese Lagereinrichtung 22 als Stift, insbesondere als Gewindestift ausgeführt, der am distalen Ende des Instruments 10 angeordnet ist. Der Gewindestift 22 kann fest am Hauptkörper 20 angebracht sein, wobei dann der gesamte Hauptkörper 20 gedreht werden muss. Vorzugsweise ist der Hauptkörper 20 daher als Hohlkörper ausgebildet, der eine als Welle 21 ausgebildete Lagereinrichtung 22 aufnehmen kann. Eine andere Lagereinrichtung 22 kann beispielsweise als Klemmvorrichtung ausgebildet sein (nicht gezeigt), bei der das Wirbelsäulenstützelement von zwei Klemmbacken in Art einer Zange gehalten wird, die dann durch eine entsprechende Mechanik gelöst werden können. Auch hier kann die Mechanik zum Lösen der Klemmbacken in einem hohlen Hauptkörper 20 aufgenommen werden.

Das Betätigungselement 24 für die Lagereinrichtung ist vorzugsweise am proximalen Ende des Instruments vorgesehen. Insbesondere ist dazu der Hauptkörper 20 mit dem Hohldurchgang ausgebildet, durch den das Betätigungselement 24 mit der Lagereinrichtung 22 verbunden werden kann und die Lagereinrichtung so betätigen kann. Wenn der Hauptkörper als Hohlkörper ausgebildet ist wird es ermöglicht, die Verbindungsmechanik zwischen Betätigungselement und Lagereinrichtung nicht auf der Außenseite des Instruments anzubringen, wodurch auf der Außenseite keine störenden Elemente vorhanden sind und die Handhabung des Instruments vereinfachen. Wie oben erwähnt ist in der bevorzugten Lösung eine Welle 21 im Hauptkörper 20 gelagert, die mit dem Gewindestift 22 am distalen Ende insbesondere einstückig verbunden ist und die am proximalen Ende einen Handgriff als Betätigungselement 24 aufweist. Vorzugsweise ist diese Welle an zumindest zwei Stellen 23a, 23b innerhalb des Hohlkörpers 20 des Hauptkörpers gelagert, bspw. in Gleitlagerbuchsen. Es ist auch vorstellbar, dass statt Gleitlagern andere Wälzlager, wie beispielsweise Nadellager, verwendet werden. Vorzugsweise befindet sich eine Lagerstelle mit einem großen Durchmesser an der proximalen Hälfte der Welle, welche sich schrittweise oder kontinuierlich bis zur Lagereinrichtung 22 am distalen Ende verjüngt. In der bevorzugten Ausführungsform ist eine dreifache schrittweise Verjüngung (reduzierung des Wellendurchmessers) vorgesehen. Gelagert wird die Lagereinrichtung einmal an der Stelle mit dem größten Durchmesser und an der Stelle mit dem kleinsten Durchmesser, wobei die Verjüngung in Richtung des distalen Endes dafür sorgt, dass das Instrument einfach in die Durchgangsbohrung des Hohlkörpers 20 eingesetzt werden kann und fest in den Lagerstellen (z.B. Lagerbuchsen) sitzt. Eine spezielle Schmierung ist nicht notwendig. Durch die kontinuierliche Verjüngung ist es sehr einfach die Welle aus dem Hauptkörper zu entfernen, indem sie einfach aus dem proximalen Ende herausgenommen wird. Ferner können die unterschiedlichen Durchmesser in der Welle auch als Anschlag verwendet werden, um die Länge, die der Gewindestift am distalen Ende aus dem Hauptkörper hervorstehen kann, zu begrenzen.

Am Instrument kann vorzugsweise auch eine Sicherung gegen das Herausfallen der Welle 21 aus dem Hauptkörper 20 vorgesehen sein. In der in den Figuren gezeigten Ausführungsform ist diese Sicherung durch ein Innengewinde 29 am proximalen Ende des Hauptkörpers 20 und ein Außengewinde 28 am größten Durchmesser der Welle 21 ausgebildet. Wenn die Welle 21 in den hohlen Hauptkörper eingesetzt wird, wird das Außengewinde durch das Innengewinde hindurch geschraubt und hat dann ein Axialspiel, kann aber nicht mehr aus dem proximalen Ende herausfallen. Solange ein Wirbelstützelement an das Lagerelement angeschraubt ist, kann das Außengewinde 28 darüber hinaus auch nicht mehr mit dem Innengewinde 29 eingreifen. Das Außengewinde 28 hat auch keine nachteilige Auswirkung auf die Lagerstelle 23b, sondern kann die Lagerfunktion auch mit Gewinde für das Instrument voll erfüllen. Andere Herausfallsicherungen können beispielsweise als eine Kappe mit Durchgangsloch ausgebildet sein, die zwischen Betätigungselement 24 und Hauptkörper 20 angeordnet ist und am Hauptkörper befestigt ist (bspw. auf den Hauptkörper gesteckt oder geschraubt). Das Durchgangsloch ist dann kleiner als der große Durchmesser der Welle 21, so dass diese nicht aus dem Hauptkörper herausfallen kann, ohne dass die Kappe zuerst vom Hauptkörper abgenommen wird. Die Sicherung muss nicht zwingend am proximalen Ende vorgesehen werden. Insbesondere die Gewindesicherung kann an einer beliebigen Stelle des Hauptkörpers vorgesehen sein, da diese insbesondere lediglich zwei aufeinander abgestimmten Gewindeabschnitte umfasst.

Mit dem Handgriff am proximalen Ende kann die Welle gedreht werden. Diese Drehung überträgt sich auf den Gewindestift am distalen Ende und dreht diesen dadurch aus dem Wirbelsäulenstützelement heraus. Insbesondere ist das Wirbelsäulenstützelement dabei durch einen Haltevorsprung 25 an einer Rotation gehindert, so dass das Instrument selbst auch dafür sorgt, dass das Wirbelsäulenstützelement sich nicht drehen kann und nicht die an das Wirbelsäulenstützelement benachbarten Wirbelsäulenelemente eine Drehung des Wirbelsäulenstützelements verhindern müssen. So wird sichergestellt, dass das Wirbelsäulenstützelement auch in seiner vorbestimmten Position bleibt, während die Lagereinrichtung vom Wirbelsäulenstützelement gelöst wird. Theoretisch ist es auch denkbar, dass der Vorsprung am Wirbelsäulenstützelement vorgesehen ist, und eine entsprechende Vertiefung am distalen Ende des Instruments. Jedoch ist es vorteilhaft, wenn der Vorsprung am Instrument vorhanden ist, da das Wirbelsäulenstützelement möglichst wenig zweckfremde vorspringende Teile aufweisen soll, also Elemente, die nicht dem Zweck der Wirbelsäulenstützung dienen.

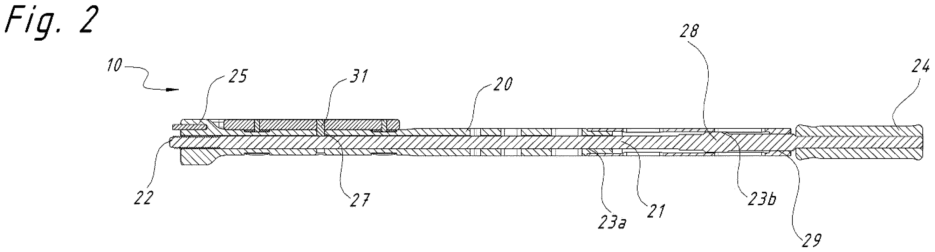

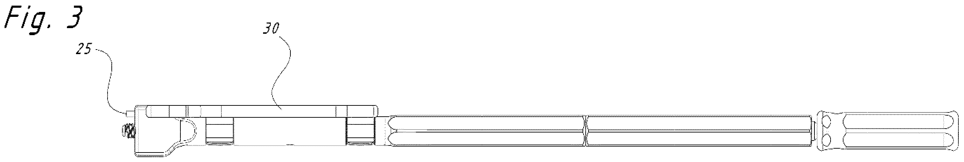

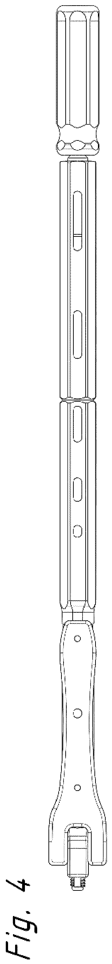

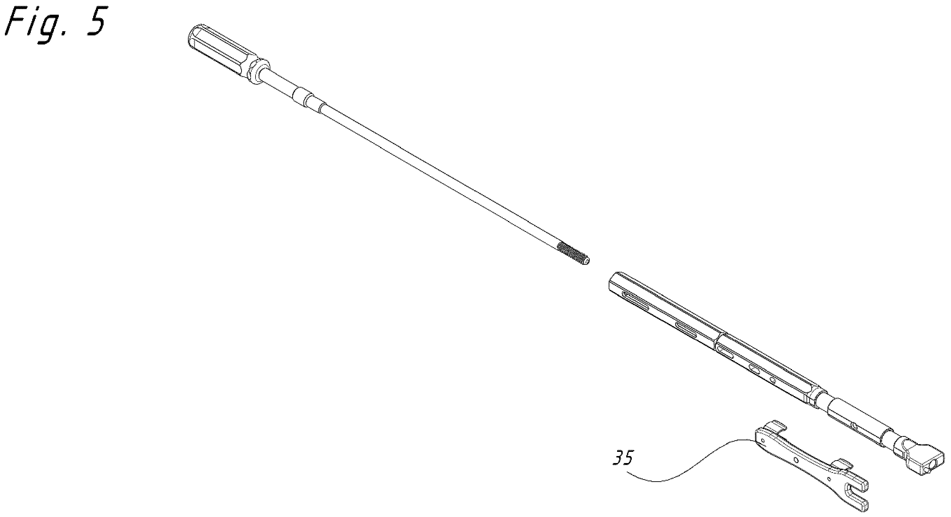

Das Instrument weist ferner einen Abstandhalter 30 auf, der die Position in Axialrichtung begrenzt, in die das Wirbelsäulenstützelement zwischen die Wirbel eingeschoben werden kann. Dazu weist der Abstandhalter 30 eine Anschlagfläche 32 auf, die dann an einen entsprechenden Abschnitt eines Wirbels anstößt. Der Abstandhalter 30 kann an einem ersten Befestigungsmittel 26 am Hauptkörper 20 befestigt werden. Dafür weist der Abstandhalter 30 ein zweites Befestigungsmittel 36 auf, das komplementär zum ersten Befestigungsmittel 26 ist. Beispielsweise kann das erste Befestigungsmittel 26 ein Stift sein und der Abstandhalter ein entsprechendes Loch aufweisen, in das der Stift eingesetzt wird (oder umgekehrt). Vorzugsweise weist jedoch der Abstandhalter 30 eine oder mehrere Federklammern 36 auf, die in entsprechende Einsetzaussparungen 26 am Hauptkörper eingeklemmt werden (oder umgekehrt). Die Federklammern 36 weisen am offenen Ende der Federn nach außen wegstehende Enden 35 auf. Dadurch wird das Aufsetzen des Abstandhalters erleichtert. Insbesondere können mehrere solche zweiten Befestigungsmittel 36 entlang des Abstandhalters vorgesehen sein. Die Befestigungsmittel legen den Abstandhalter zumindest in Axialrichtung zur proximalen Seite des Instruments fest. Beispielsweise könnte statt einem Abstandhalter, wie er in den Figuren 1 bis 4 gezeigt ist, auch ein Abstandhalter vorgesehen sein, der wie der Verdrehsicherungsstift für das Wirbelsäulenstützelement im Instrument gelagert ist. D.h., das Instrument weist an einer Stelle, beispielsweise in einer Entfernung ähnlich dem Verdrehsicherungsstift 25 zur Mittelachse aber senkrecht zur Lage des Wirbelkörperstützelements, eine Aussparung auf, in die ein Abstandhaltestift (nicht gezeigt) eingesetzt werden kann. Dazu müsste das distale Ende des Instruments 10, das in Figur 5 einen rechteckigen Querschnitt hat mit einem kreisförmigen Querschnitt oder zumindest kreuzförmig ausgebildet sein. So können unterschiedliche Abstandhalterstifte eingesetzt werden um das instrument für verschiedene Wirbelsäulenstützelemente verwendbar zu machen. Oder der Abstandhalterstift wird ebenfalls mit einem Gewinde ausgeführt, und so als einstellbarer Abstandhalter ausgebildet. Alternativ kann der Abstandhalter aber auch nicht lösbar auf dem Hauptkörper montiert sein. In diesem Fall muss der Abstandhalter dann aber verstellbar auf dem Hauptkörper befestigt sein, bspw. mittels einem Gewindering rotationsfest zum Hauptkörper auf dem Hauptkörper gelagert sein, so dass durch Drehen des Gewinderings der Abstandhalter sich entlang der Längsachse bewegt.

Für eine steckbare Verbindung weist der Abstandhalter die schon oben genannten Klemmfedern 36 auf, die dann um den Hauptkörper 20 an entsprechenden Eingriffsabschnitten 26 aufgesteckt werden und so am Hauptkörper 20 befestigt werden. Vorzugsweise weist der Abstandhalter als Verdrehsicherung einen Sicherungsstift 31 auf, der in eine entsprechende Aussparung 27 des Hauptkörpers 20 eingreift. Alternativ kann auch der Hauptkörper 20 einen Sicherungsvorsprung aufweisen, der in eine entsprechende Aussparung des Abstandhalters 30 eingreift. in einer weiteren Ausgestaltung kann die Verdrehsicherung auch nur als glatte Fläche ausgebildet sein, an die der Abstandhalter mit einer eigenen Fläche anliegt und so das Verdrehen des Abstandhalters erschwert. Der Abstandhalter 30 ist vorzugsweise als sich entlang der Längsachse erstreckender Körper ausgebildet, wobei der Anschlag 32 vorzugsweise als zur Längsachse im Wesentlichen senkrechte Fläche vorgesehen ist. In der bevorzugten Ausführungsform ist der Abstandhalter gabelförmig ausgebildet, mit zwei zur Längsachse des Instruments symmetrischen und im Wesentlichen parallelen, vorstehenden "Gabelzinken" mit eigenen Anschlagsflächen 32. Diese beiden sind vorzugsweise gleich oder weiter voneinander entfernt, als die Dicke des eingesetzten Wirbelsäulenstützelements.

Die Verdrehsicherungen 27 (Aussparungen, Stifte, glatte Flächen) können am Hauptkörper umfänglich auch mehrfach vorgesehen sein, so dass der Abstandhalter an verschiedenen Positionen in Umfangsrichtung festgesetzt werden kann.

Am Hauptkörper 20 (oder am Abstandhalter 30) können mehrere Befestigungsmittel vorgesehen sein, so dass ein Abstandhalter auch an verschiedenen Stellen des Hauptkörpers aufgesetzt werden kann. Konkret für die in den Figuren gezeigte Ausführungsform können beispielsweise die Eingriffsabschnitte entlang der Axialrichtung wesentlich breiter ausgeführt werden und der Verdrehsicherungsstift gleichzeitig auch die Axialrichtung festlegen. Dafür sind dann entlang des Hauptkörpers mehrere entsprechende Eingriffsaussparungen für den Verdrehsicherungsstift nicht nur in Umfangsrichtung, sondern auch entlang der Axialrichtung vorgesehen. So legt dann der Verdrehsicherungsstift auch die Axialposition des Abstandhalters fest, und die beiden Klemmfedern 36 halten den Abstandhalter 30 am Hauptkörper 20 lösbar fest.

Im Einsatz wird ein Wirbelsäulenstützelement an der Lagereinrichtung befestigt, dann eingesetzt, so dass der Abstandhalter mit seinem Anschlag an den entsprechenden Wirbelknochen anschlägt und dann das Betätigungselement zum Freilassen des Wirbelsäulenstützelements betätigt. Konkret für die gezeigte Ausführungsform wird bspw. ein Cage auf das Verdrehsicherungselement 25 gesteckt, dann mittels des Betätigungselements 24 und der Durchgangswelle 21 der Gewindestift 22 in den Cage eingedreht, der Abstandhalter 30 auf dem Hauptkörper 20 in der richtigen Position platziert und dann der Cage eingesetzt. Wenn der Abstandhalter 30 anschlägt, ist die richtige Position für den Cage angezeigt und mittels des Betätigungselements 24 wird der Gewindestift 22 aus dem Cage ausgeschraubt und das Instrument vom Patienten weg aus dem Cage herausgenommen.

## Patentansprüche

1. Instrument (10) zum Implantieren eines Wirbelsäulenstützelements umfassend:
einen Hauptkörper (20) mit einem Griffabschnitt zum Halten und Führen des Instruments (10) und mit einer Lagereinrichtung (22) am distalen Ende des Hauptkörpers (20) zum Lagern des Wirbelsäulenstützelements, wobei der Hauptkörper (20) ein erstes Befestigungsmittel (26) aufweist;
einen Abstandhalter (30) mit zumindest einem Anschlag (32) und einem zum ersten Befestigungsmittel (26) komplementären zweiten Befestigungsmittel (36);
wobei die Befestigungsmittel (26, 36) voneinander lösbar sind und den Abstandhalter (30) zumindest in proximaler Axialrichtung festlegen
**dadurch gekennzeichnet, dass**
die Lagereinrichtung (22) sich durch den gesamten Hauptkörper (20) erstreckt und die Lagereinrichtung als Welle ausgebildet ist, die sich schrittweise oder kontinuierlich vom proximalen Ende bis zum distalen Ende verjüngt.

2. Instrument (10) nach Anspruch 1, bei dem die Befestigungsmittel (26, 36) als Steckmittel ausgebildet sind.

3. Instrument (10) nach Anspruch 1 oder 2, bei dem der Abstandhalter (30) gabelförmig mit zumindest zwei Gabelästen ausgebildet.

4. Instrument (10) nach einem der vorhergehenden Ansprüche, bei dem der Abstandhalter seitlich am, auf oder unter dem Hauptkörper (20) angebracht ist.

5. Instrument (10) nach einem der vorhergehenden Ansprüche, bei dem der Hauptkörper (20) und der Abstandhalter (30) jeweils zumindest zwei Befestigungsmittel (26, 36) aufweisen.

6. Instrument (10) nach einem der vorhergehenden Ansprüche, bei dem das zumindest eine erste und zweite Befestigungsmittel (26, 36) als Federklemme und als Eingriffsabschnitt ausgebildet sind, in den die Federklemme eingreift.

7. Instrument (10) nach einem der vorhergehenden Ansprüche, bei dem mehr erste als zweite Befestigungsmittel (26, 36) oder mehr zweite als erste Befestigungsmittel (26, 36) vorgesehen sind.

8. Instrument (10) nach einem der vorhergehenden Ansprüche, bei dem der Hauptkörper (20) stabförmig ausgebildet ist.

9. Instrument (10) nach einem der vorhergehenden Ansprüche, bei dem die Lagereinrichtung (22) als Stift mit einem Gewinde am distalen Ende ausgebildet ist, auf den das Wirbelsäulenstützelement aufschraubbar ist.

10. Instrument (10) nach einem der vorhergehenden Ansprüche, bei dem sich die Lagerleinrichtung (22) am proximalen Ende betätigbar ist.

11. Instrument (10) nach Anspruch 10, bei dem die Lagereinrichtung (22) lösbar in dem Hauptkörper (20) gelagert ist.

12. Instrument (10) nach einen der vorherigen Ansprüchen, **dadurch gekennzeichnet, dass** zwischen Hauptkörper (20) und Wirbelsäulenstützelement eine Rotationshemmungselement (25) vorgesehen ist.

13. Instrument (10) nach einen der vorherigen Ansprüchen, **dadurch gekennzeichnet, dass** der Abstandhalter (30) am Hauptkörper (20) rotationsgesichert befestigt ist.

14. Instrument (10) zum Implantieren eines Wirbelsäulenstützelements umfassend:
einen Hauptkörper (20) mit einem Griffabschnitt zum Halten und Führen des Instruments (10) und mit einer Lagereinrichtung (22) am distalen Ende des Hauptkörpers (20) zum Lagern des Wirbelsäulenstützelements;
einen Abstandhalter (30) mit zumindest einem Anschlag (32);
wobei der Abstandhalter (30) in Axialrichtung verstellbar am Hauptkörper (20) befestigt ist,
**dadurch gekennzeichnet, dass**
die Lagereinrichtung (22) sich durch den gesamten Hauptkörper (20) erstreckt und die Lagereinrichtung als Welle ausgebildet ist, die sich schrittweise oder kontinuierlich vom proximalen Ende bis zum distalen Ende verjüngt.

15. Instrument (10) nach Anspruch 14, mit den Merkmalen von einem oder mehreren der Ansprüche 3, 4 und 8 bis 13.

## Claims

1. An instrument (10) for implanting a spine support member comprising:
a main body (20) having a handle portion for holding and guiding the instrument (10) and bearing means (22) at the distal end of the main body (20) for supporting the spine support member, the main body (20) having a first attachment means (26);
a spacer (30) having at least one stop (32) and a second fastening means (36) complementary to the first fastening means (26);
wherein the fastening means (26, 36) are detachable from each other and fix the spacer (30) at least in the proximal axial direction
**characterized in that**
the bearing means (22) extends through the entire main body (20) and the bearing means is formed as a shaft which tapers stepwise or continuously from the proximal end to the distal end.

2. Instrument (10) according to claim 1, wherein the fastening means (26, 36) are designed as plug-in means.

3. Instrument (10) according to claim 1 or 2, wherein the spacer (30) is designed forkshaped with at least two fork branches.

4. Instrument (10) according to any one of the preceding claims, wherein the spacer is mounted laterally on, on or under the main body (20).

5. Instrument (10) according to any one of the preceding claims, wherein the main body (20) and the spacer (30) each have at least two fastening means (26, 36).

6. Instrument (10) according to any one of the preceding claims, wherein the at least one first and second fastening means (26, 36) are designed as a spring clamp and engagement portion in which the spring clamp engages.

7. Instrument (10) according to any one of the preceding claims, wherein more first than second fastening means (26, 36) or more second than first fastening means (26, 36) are provided.

8. Instrument (10) according to any one of the preceding claims, wherein the main body (20) is rod-shaped.

9. Instrument (10) according to any one of the preceding claims, wherein the bearing means (22) is designed as a pin with a thread at the distal end, on which the spine support member can be screwed.

10. Instrument (10) according to any one of the preceding claims, wherein the bearing device (22) is actuated at the proximal end.

11. Instrument (10) according to claim 10, wherein the bearing means (22) is detachably mounted in the main body (20).

12. Instrument (10) according to one of the preceding claims, **characterized in that** between the main body (20) and the spine support member, a rotation inhibiting element (25) is provided.

13. Instrument (10) according to one of the preceding claims, **characterized in that** the spacer (30) on the main body (20) is mounted so that it is secured against rotation.

14. An instrument (10) for implanting a spine support member comprising:
a main body (20) having a handle portion for holding and guiding the instrument (10) and bearing means (22) at the distal end of the main body (20) for supporting the spine support member;
a spacer (30) having at least one stop (32);
wherein the spacer (30) is adjustably mounted on the main body (20) in an axial direction
**characterized in that**
the bearing means (22) extends through the entire main body (20) and the bearing means is formed as a shaft which tapers stepwise or continuously from the proximal end to the distal end.

15. Instrument (10) according to claim 14, having the features of one or more of claims 3, 4 and 8 to 13.

## Revendications

1. Instrument (10) pour implanter un élément de support de la colonne vertébrale comprenant:
un corps principal (20) ayant une partie de poignée pour tenir et guider l'instrument (10) et un moyen de palier (22) au niveau de l'extrémité distale du corps principal (20) pour supporter l'élément de support de la colonne vertébrale, le corps principal (20) comportant un premier moyen de fixation (26);
une entretoise (30) ayant au moins une butée (32) et un second moyen de fixation (36) complémentaire du premier moyen de fixation (26);
dans lequel les moyens de fixation (26, 36) sont détachables l'un de l'autre et fixent l'entretoise (30) au moins dans la direction axiale proximale
**caractérisé en ce que**
le moyen de palier (22) s'étend à travers tout le corps principal (20) et le moyen de palier est conçu comme un arbre qui se rétrécit progressivement ou continuellement de l'extrémité proximale à l'extrémité distale.

2. Instrument (10) selon la revendication 1, dans lequel les moyens de fixation (26, 36) sont conçus comme des moyens d'enfichage.

3. Instrument (10) selon la revendication 1 ou 2, dans lequel l'entretoise (30) est en forme de fourche avec au moins deux branches de fourche.

4. Instrument (10) selon l'une quelconque des revendications précédentes, dans lequel l'entretoise est montée latéralement sur, sur ou sous le corps principal (20).

5. Instrument (10) selon l'une quelconque des revendications précédentes, dans lequel le corps principal (20) et l'entretoise (30) comportent chacun au moins deux moyens de fixation (26, 36).

6. Instrument (10) selon l'une quelconque des revendications précédentes, dans lequel les au moins un premier et second moyens de fixation (26, 36) sont conçus comme une pince à ressort et une partie d'engagement dans laquelle s'engage la pince à ressort.

7. Instrument (10) selon l'une quelconque des revendications précédentes, dans lequel plus de premiers que seconds moyen de fixation (26, 36), ou plus de deuxièmes que premiers moyen de fixation (26, 36) sont prévus.

8. Instrument (10) selon l'une quelconque des revendications précédentes, dans lequel le corps principal (20) est en forme de tige.

9. Instrument (10) selon l'une quelconque des revendications précédentes, dans lequel le moyen de palier (22) est conçu comme une broche avec un filetage à l'extrémité distale, sur lequel l'élément de support de la colonne vertébrale peut être vissé.

10. Instrument (10) selon l'une quelconque des revendications précédentes, dans lequel le moyen de palier (22) est actionné à l'extrémité proximale.

11. Instrument (10) selon la revendication 10, dans lequel le moyen de palier (22) est montés de manière amovible dans le corps principal (20).

12. Instrument (10) selon l'une des revendications précédentes, **caractérisé en ce qu'**un élément empêchant la rotation (25) est prévu entre le corps principal (20) et l'élément de support de la colonne vertébrale.

13. Instrument (10) selon l'une des revendications précédentes, **caractérisé en ce que** l'entretoise (30) sur le corps principal (20) est bloquée en rotation.

14. Instrument (10) pour implanter un élément de support de la colonne vertébrale comprenant:
un corps principal (20) ayant une partie de poignée pour tenir et guider l'instrument (10) et un moyen de palier (22) au niveau de l'extrémité distale du corps principal (20) pour supporter l'élément de support de la colonne vertébrale;
une entretoise (30) ayant au moins une butée (32);
dans lequel l'entretoise (30) est montée de manière réglable dans une direction axiale sur le corps principal (20)
**caractérisé en ce que**
le moyen de palier (22) s'étend à travers tout le corps principal (20) et le moyen de palier est conçu comme un arbre qui se rétrécit progressivement ou continuellement de l'extrémité proximale à l'extrémité distale.

15. Instrument (10) selon la revendication 14, présentant les caractéristiques d'une ou de plusieurs des revendications 3, 4 et 8 à 13.
